# EUROPÄISCHE PATENTANMELDUNG

(11) **EP 3 629 339 A1**
(43) Veröffentlichungstag der Anmeldung: **01.04.2020**
(21) Anmeldenummer: 19191202.1
(22) Anmeldetag: 12.08.2019
(51) Int. Cl.: G16H 30/40, A61B 6/00, A61N 5/10, G06T 7/00, G06T 11/00

(54) **BEREITSTELLEN EINES ANNOTIERTEN MEDIZINISCHEN BILDDATENSATZES FÜR EINE RADIOTHERAPIEPLANUNG EINES PATIENTEN**

(30) Priorität: 25.09.2018 DE 102018216370
(71) Anmelder: Siemens Healthcare GmbH, 91052 Erlangen (DE)
(72) Erfinder: Ritter, André, 91077 Neunkirchen am Brand (DE)

(57) **Zusammenfassung**

Die Erfindung betrifft ein Verfahren für ein Bereitstellen eines annotierten medizinischen Bilddatensatzes für eine Radiotherapieplanung eines Patienten mit folgenden Schritten:
- Bereitstellen einer Planungsbildvorschrift für die Radiotherapieplanung, welche eine Aufnahmevorschrift und eine erste Rekonstruktionsvorschrift aufweist,
- Abrufen einer Annotierungsvorschrift aus einer Speichereinheit, wobei die Annotierungsvorschrift Annotierungsinstruktionen und eine zweite Rekonstruktionsvorschrift aufweist,
- Erfassen von Rohdaten des Patienten mittels einer medizinischen Bildgebungseinheit gemäß der Aufnahmevorschrift,
- Rekonstruieren eines ersten medizinischen Bilddatensatzes gemäß der ersten Rekonstruktionsvorschrift unter Verwendung der Rohdaten,
- Rekonstruieren eines zweiten medizinischen Bilddatensatzes gemäß der zweiten Rekonstruktionsvorschrift unter Verwendung der Rohdaten,
- Annotieren des zweiten medizinischen Bilddatensatzes gemäß den Annotierungsinstruktionen, wobei Annotierungsbildinformationen ermittelt werden,
- Kombinieren des ersten medizinischen Bilddatensatzes mit den Annotierungsbildinformationen, wobei ein annotierter medizinischer Bilddatensatz bereitgestellt wird.

## Beschreibung

Die Erfindung betrifft ein Verfahren für ein Bereitstellen eines annotierten medizinischen Bilddatensatzes für eine Radiotherapieplanung eines Patienten, eine zugehörige medizinische Bildgebungseinheit und ein zugehöriges Computerprogrammprodukt.

Eine Radiotherapie eines Patienten umfasst üblicherweise eine gezielte Bestrahlung von einem Tumor, insbesondere zu einer Behandlung von Tumorerkrankungen. Die Radiotherapie erfolgt typischerweise gemäß einem Bestrahlungsmuster, welches sehr komplex sein kann.

Eine Radiotherapieplanung umfasst insbesondere ein Festlegen des Bestrahlungsmusters und erfordert typischerweise einen annotierten medizinischen Bilddatensatz, welcher ein Planungsbild und Annotierungsbildinformationen in einem einzigen medizinischen Bild oder in zwei separaten medizinischen Bildern aufweist. Ein Bereitstellen des annotierten medizinischen Bilddatensatzes ist insbesondere für einen Nutzer einer medizinischen Bildgebungseinheit aufwändig und/oder zeitintensiv. Eine erste Anforderung, dass der annotierte medizinische Bilddatensatz das Planungsbild aufweist, kann einer zweiten Anforderung, dass der annotierte medizinische Bilddatensatz ebenfalls die Annotierungsbildinformationen aufweist, entgegenstehen. Daher kann es für das Bereitstellen des annotierten medizinischen Bilddatensatzes nötig sein, dass bei aufeinanderfolgenden bildgebenden Messungen mehrmals Rohdaten mittels der medizinischen Bildgebungseinheit erfasst werden, um die erste Anforderung und die zweite Anforderung erfüllen zu können.

Der Erfindung liegt die Aufgabe zu Grunde, ein Verfahren für ein Bereitstellen eines annotierten medizinischen Bilddatensatzes für eine Radiotherapieplanung eines Patienten, eine zugehörige medizinische Bildgebungseinheit und ein zugehöriges Computerprogrammprodukt anzugeben, bei welchen das Planungsbild und die Annotierungsbildinformationen nach einer einzigen bildgebenden Messung bereitgestellt werden kann.

Die Aufgabe wird durch die Merkmale der unabhängigen Ansprüche gelöst. Vorteilhafte Ausgestaltungen sind in den Unteransprüchen beschrieben.

Das erfindungsgemäße Verfahren für ein Bereitstellen eines annotierten medizinischen Bilddatensatzes für eine Radiotherapieplanung eines Patienten weist folgende Schritte auf:
- Bereitstellen einer Planungsbildvorschrift für die Radiotherapieplanung, welche eine Aufnahmevorschrift und eine erste Rekonstruktionsvorschrift aufweist,
- Abrufen einer Annotierungsvorschrift aus einer Speichereinheit, wobei die Annotierungsvorschrift Annotierungsinstruktionen und eine zweite Rekonstruktionsvorschrift aufweist und wobei sich die zweite Rekonstruktionsvorschrift von der ersten Rekonstruktionsvorschrift unterscheidet,
- Erfassen von Rohdaten des Patienten mittels einer medizinischen Bildgebungseinheit gemäß der Aufnahmevorschrift,
- Rekonstruieren eines ersten medizinischen Bilddatensatzes gemäß der ersten Rekonstruktionsvorschrift unter Verwendung der Rohdaten,
- Rekonstruieren eines zweiten medizinischen Bilddatensatzes gemäß der zweiten Rekonstruktionsvorschrift unter Verwendung der Rohdaten,
- Annotieren des zweiten medizinischen Bilddatensatzes gemäß den Annotierungsinstruktionen, wobei Annotierungsbildinformationen ermittelt werden,
- Kombinieren des ersten medizinischen Bilddatensatzes mit den Annotierungsbildinformationen, wobei ein annotierter medizinischer Bilddatensatz erzeugt wird, und
- Bereitstellen des annotierten medizinischen Bilddatensatzes für die Radiotherapieplanung des Patienten.

Das Verfahren für das Bereitstellen des annotierten medizinischen Bilddatensatzes für die Radiotherapieplanung des Patienten weist insbesondere folgende Vorteile auf:
Das Abrufen der Annotierungsvorschrift aus der Speichereinheit ermöglicht vorzugsweise, dass der annotierte medizinische Bilddatensatz ein Planungsbild und Annotierungsbildinformationen aufweist, wobei ein Nutzer der medizinischen Bildgebungseinheit vorzugsweise lediglich die Planungsbildvorschrift bereitstellen kann. In anderen Worten fällt vorzugsweise für den Nutzer der medizinischen Bildgebungseinheit Arbeitsaufwand dadurch weg, dass die Annotierungsvorschrift aus der Speichereinheit abgerufen wird, wodurch typischerweise Kosten eingespart werden können. Alternativ oder zusätzlich kann eine Fehlerquelle dadurch wegfallen, dass die Annotierungsvorschrift aus der Speichereinheit abgerufen wird und insbesondere nicht von dem Nutzer bereitgestellt wird. Insbesondere ein automatisches Abrufen der Annotierungsvorschrift aus der Speichereinheit ohne Nutzerinteraktion ist besonders vorteilhaft.

Vorteilhafterweise weist der annotierte medizinische Bilddatensatz in hoher Bildqualität das Planungsbild und die Annotierungsbildinformationen auf, wobei idealerweise das Festlegen des Bestrahlungsmusters der Radiotherapieplanung in hoher Qualität erfolgen kann und/oder ein Widerspruch vorteilhafterweise aufgelöst werden kann. Der Widerspruch lautet insbesondere, dass eine erste Anforderung, wobei der annotierte medizinische Bilddatensatz das Planungsbild, insbesondere in hoher Bildqualität, aufweist, einer zweiten Anforderung entgegensteht, wobei der annotierte medizinische Bilddatensatz ebenfalls die Annotierungsbildinformationen, insbesondere in hoher Bildqualität, aufweist. Vorzugsweise kann der annotierte medizinische Bilddatensatz das Planungsbild und die Annotierungsbildinformationen aufweisen, ohne einen Bildeindruck des annotierten medizinischen Bilddatensatzes, insbesondere des Planungsbildes, für den Nutzer zu verfälschen.

Durch das Bereitstellen des annotierten medizinischen Bilddatensatzes, insbesondere der Annotierungsbildinformationen gemeinsam mit dem Planungsbild, kann vorzugsweise weiterer Arbeitsaufwand für den Nutzer eingespart werden, weil die Annotierungsbildinformationen bereits verfügbar sind und insbesondere nicht durch den Nutzer manuell bereitgestellt werden müssen. Für das manuelle Bereitstellen wird nämlich üblicherweise ein herkömmlicher medizinischer Bilddatensatz verwendet, welcher beispielsweise das Planungsbild, aber nicht gleichzeitig die Annotierungsbildinformationen in hoher Bildqualität aufweisen kann, außer wenn insbesondere mehrere bildgebende Messungen durchgeführt werden. Vorzugsweise ist eine mittlere Bildqualität des annotierten medizinischen Bilddatensatzes höher als eine mittlere Bildqualität des herkömmlichen medizinischen Bilddatensatzes. Weil der annotierte medizinische Bilddatensatz insbesondere mittels der bei einer einzigen bildgebenden Messung erfassten Rohdaten des Patienten bereitgestellt werden, kann vorzugsweise auf weitere bildgebende Messungen, insbesondere für das Bereitstellen des Planungsbilds und der Annotierungsbildinformationen, verzichtet werden, was mit einer Reduktion an kostspieliger Untersuchungsdauer und/oder Röntgenstrahlendosis einhergehen kann.

Ein weiterer Vorteil kann sein, dass insbesondere das Bereitstellen der Annotierungsbildinformationen standardisiert und/oder in Hinblick auf Referenzwerte vergleichbar ist, weil die Annotierungsvorschrift aus der Speichereinheit abgerufen wird. Vorzugsweise sind die Annotierungsbildinformationen unabhängig von der bereitgestellten Planungsbildvorschrift, insbesondere von der ersten Rekonstruktionsvorschrift. Beispielsweise kann für mehrere verschiedene Patienten die gleiche Annotierungsvorschrift, aber unterschiedliche Planungsbildvorschriften verwendet werden. Dadurch kann insbesondere in Hinblick auf Annotierungsbildinformationen der mehreren verschiedenen Patienten eine hohe Vergleichbarkeit gewährleistet sein.

Der bereitgestellte annotierte medizinische Bilddatensatz ist insbesondere für eine automatisierte Radiotherapieplanung des Patienten vorteilhaft, weil die automatisierte Radiotherapieplanung üblicherweise standardisierte Konturierungs- und/oder Segmentierungsverfahren verwendet, was insbesondere mittels der standardisierten Annotierungsbildinformationen ermöglicht wird. Alternativ oder zusätzlich kann vorteilhafterweise eine klinisch-relevante Struktur des Patienten präziser mittels des annotierten medizinischen Bilddatensatzes bereitgestellt werden als mittels des herkömmlichen medizinischen Bilddatensatzes. Vorzugsweise ist das mittels des annotierten medizinischen Bilddatensatzes festgelegte Bestrahlungsmuster präziser und damit insbesondere für den Patienten in Hinblick auf eine Prognose und/oder einen Behandlungserfolg und/oder eine Strahlenbelastung vorteilhafter.

Bei verschiedenen Radiotherapieverfahren, welche für die Radiotherapie des Patienten geeignet sind, kommen insbesondere mehrere Arten an ionisierender Strahlung zum Einsatz, beispielsweise Gammastrahlung, insbesondere als Photonen-Strahlung, Betastrahlung, insbesondere als Elektronen-Strahlung, und/oder in einer Partikelradiotherapie Hadronenbasierte Strahlung, insbesondere als Protonen-, Neutronen-, He-Ionen-, Alpha- und/oder Kohlenstoff-Ionen-Strahlung. Die verschiedenen Radiotherapieverfahren können üblicherweise in interne Radiotherapieverfahren oder in externe Radiotherapieverfahren unterteilt werden. Bei den externen Radiotherapieverfahren wird die ionisierende Strahlung typischerweise außerhalb des Patienten erzeugt, beispielsweise mittels radioaktiver Isotope und/oder Teilchenbeschleunigeranlagen. Bei den internen Radiotherapieverfahren wird üblicherweise eine Strahlenquelle zur Erzeugung der ionisierenden Strahlung in den Patienten, beispielsweise mittels eines chirurgischen und/oder minimal invasiven Eingriffs, eingebracht.

Die Radiotherapieplanung umfasst insbesondere ein Festlegen des Bestrahlungsmusters. Für die Radiotherapie wird typischerweise bei der Radiotherapieplanung das Bestrahlungsmuster festgelegt. Das Bestrahlungsmuster weist insbesondere eine Simulation über eine ortsaufgelöste Strahlendosis auf, welche insbesondere eine Vorhersage über die Verteilung der ionisierenden Strahlung in dem Patienten aufweist. Das Bestrahlungsmuster beschreibt insbesondere wie die ionisierende Strahlung mittels Strahlformungselementen, insbesondere mittels Multi-Leaf-Kollimatoren, geformt und/oder aus mehreren Einstrahlrichtungen in den Patienten eingestrahlt wird. Das Bestrahlungsmuster weist typischerweise eine Zieldosisverteilung und/oder eine Maximalstrahlendosisverteilung auf. Die Zieldosisverteilung und/oder die Maximalstrahlendosisverteilung werden insbesondere mittels Dosis-Volumen-Histogramme, einem Dosismittelwert, einem Dosisminimum und/oder einem Dosismaximum bestimmt, wobei insbesondere das Planungsbild und die Annotierungsbildinformationen als Eingangsparameter beim Bestimmen eingehen.

Die Zieldosisverteilung definiert insbesondere eine Mindeststrahlendosis in einem klinisch definierten Zielvolumen, wobei insbesondere die Annotierungsbildinformationen ein Abgrenzen des klinisch definierten Zielvolumens ermöglichen. Das Zielvolumen weist insbesondere einen zu behandelnden und/oder zu bestrahlenden Tumor des Patienten auf. Die Maximalstrahlendosisverteilung definiert insbesondere eine Maximalstrahlendosis außerhalb des Zielvolumens, insbesondere in einem gesunden Gewebe, beispielsweise in einem Risikoorgan. Die Maximalstrahlendosis kann für verschiedene Risikoorgane variieren. Die Radiotherapie erfolgt vorteilhafterweise derart, dass gemäß dem Bestrahlungsmuster die Zieldosisverteilung im Patienten erreicht wird und die Maximalstrahlendosisverteilung nicht überschritten wird. Vorzugsweise ermöglichen einige der verschiedenen Radiotherapieverfahren, dass die Zieldosisverteilung im Vergleich zur Maximalstrahlendosisverteilung um einen Faktor 10 bis 100 höher ist.

Das Festlegen des Bestrahlungsmusters umfasst insbesondere ein Bestimmen der Zieldosisverteilung, beispielsweise der Mindeststrahlendosis in dem Zielvolumen, und/oder der Maximalstrahlendosisverteilung, insbesondere der Maximalstrahlendosis außerhalb des Zielvolumens. Das Festlegen des Bestrahlungsmusters umfasst insbesondere ein Durchführen der Simulation über die ortsaufgelöste Strahlendosis, wobei beispielsweise eine Monte-Carlo-Simulation durchgeführt wird. Das Festlegen des Bestrahlungsmusters erfolgt üblicherweise in einem Radiotherapieplanungssystem nach dem Bereitstellen des annotierten medizinischen Bilddatensatzes. Das Radiotherapieplanungssystem kann ein Teil der medizinischen Bildgebungseinheit sein oder, insbesondere über ein Radiologieinformationssystem und/oder ein PACS-Bildarchivierungssystem, mit der medizinischen Bildgebungseinheit verbunden sein und/oder mittels der Recheneinheit bereitgestellt werden. Der Nutzer legt das Bestrahlungsmuster üblicherweise gemäß Vorgaben eines den Patienten behandelnden Radioonkologen fest. Alternativ oder zusätzlich kann das Bestrahlungsmuster bei der automatisierten Radiotherapieplanung festgelegt werden.

Das Festlegen des Bestrahlungsmusters erfordert üblicherweise, dass der annotierte medizinische Bilddatensatz das Planungsbild und die Annotierungsbildinformationen aufweist. Der annotierte medizinische Bilddatensatz ermöglicht insbesondere ein vorteilhaftes Festlegen des Bestrahlungsmusters bei der Radiotherapieplanung. Für das Festlegen des Bestrahlungsmusters werden insbesondere das Planungsbild und die Annotierungsbildinformationen gemeinsam bereitgestellt, weil dadurch insbesondere Materialeigenschaften, beispielsweise im Zielvolumen, und/oder das Zielvolumen berücksichtigt werden können.

Das Planungsbild bildet vorzugsweise die Materialeigenschaften ab, welche für die Simulation über die ortsaufgelöste Strahlendosis verwendet werden können, da die ortsaufgelöste Strahlendosis üblicherweise von den Materialeigenschaften abhängt. Die ortsaufgelöste Strahlendosis kann insbesondere mittels des Planungsbildes berechnet werden. Die Materialeigenschaften umfassen typischerweise physikalische Materialeigenschaften des Patienten. Das Planungsbild ermöglicht insbesondere ein Umrechnen von Pixelwerten des annotierten medizinischen Bilddatensatzes in die Materialeigenschaften für das Berechnen der ortsaufgelösten Strahlendosis. Das Umrechnen der Pixelwerte des annotierten medizinischen Bilddatensatzes erfolgt insbesondere gemäß einer Umrechnungstabelle. Die Pixelwerte können Bildwerten entsprechen. Das Planungsbild und/oder die Annotierungsbildinformationen sind insbesondere in den Pixelwerten des medizinischen Bilddatensatzes abgebildet. Die Pixelwerte des medizinischen Bilddatensatzes können beispielsweise gemäß einer Hounsfield-Unit und/oder weiteren Bildwertskalen skaliert sein.

Die Annotierungsbildinformationen ermöglichen vorteilhafterweise ein Festlegen des Zielvolumens bei der Radiotherapieplanung. Vorzugsweise können mittels des annotierten medizinischen Bilddatensatzes das Zielvolumen und/oder das Gewebe außerhalb des Zielvolumens insbesondere durch den Nutzer konturiert werden. Die Annotierungsbildinformationen können alternativ oder zusätzlich einen Fremdkörper, insbesondere ein Implantat, in dem Patienten und/oder ein gesamtes Patientenvolumen abbilden. Die Kontur des Zielvolumens und/oder des Gewebes außerhalb des Zielvolumens und/oder des Fremdkörpers kann beispielsweise zumindest eine Volumenaußengrenze und/oder eine anatomische Landmarke aufweisen. Die Annotierungsbildinformationen weisen insbesondere die Kontur und/oder die zumindest eine Volumenaußengrenze auf.

Das Bereitstellen der Planungsbildvorschrift für die Radiotherapieplanung kann beispielsweise durch den Nutzer der medizinischen Bildgebungseinheit erfolgen. Die medizinische Bildgebungseinheit kann beispielsweise ein Anzeigeeinheit und/oder Eingabemittel aufweisen, mittels welchen der Nutzer die Planungsbildvorschrift bereitstellt. Die Planungsbildvorschrift kann insbesondere in einem Arbeitsspeicher und/oder in einem Zwischenspeicher der medizinischen Bildgebungseinheit zwischengespeichert werden. Die Planungsbildvorschrift wird insbesondere nicht in der Speichereinheit gespeichert. Grundsätzlich ist es denkbar, dass die Annotierungsvorschrift aus der Speichereinheit abgerufen und in dem Arbeitsspeicher und/oder in dem Zwischenspeicher gemeinsam mit der Planungsbildvorschrift zwischengespeichert wird. Für das Erfassen der Rohdaten kann insbesondere die medizinische Bildgebungseinheit die Planungsbildvorschrift, insbesondere die Aufnahmevorschrift, aus dem Arbeitsspeicher und/oder dem Zwischenspeicher abrufen. Eine Recheneinheit der medizinischen Bildgebungseinheit kann insbesondere für das Rekonstruieren des ersten medizinischen Bilddatensatzes und/oder des zweiten medizinischen Bilddatensatzes die Planungsbildvorschrift, insbesondere die erste Rekonstruktionsvorschrift, und/oder die Annotierungsvorschrift, insbesondere die zweite Rekonstruktionsvorschrift, aus dem Arbeitsspeicher und/oder dem Zwischenspeicher abrufen. Die Recheneinheit der medizinischen Bildgebungseinheit kann insbesondere für das Annotieren des zweiten medizinischen Bilddatensatzes die Annotierungsvorschrift, insbesondere die Annotierungsinstruktionen, aus dem Arbeitsspeicher und/oder dem Zwischenspeicher abrufen. Grundsätzlich ist es denkbar, dass die Recheneinheit die Annotierungsvorschrift direkt aus der Speichereinheit abruft, ohne dass die Annotierungsvorschrift in dem Arbeitsspeicher und/oder dem Zwischenspeicher zwischengespeichert wird.

Die Annotierungsvorschrift wird insbesondere nicht von dem Nutzer mittels der Anzeigeeinheit und/oder Eingabemittel bereitgestellt. In anderen Worten umfasst das Bereitstellen der Planungsbildvorschrift typischerweise nicht ein Bereitstellen der Annotierungsvorschrift. Das Abrufen der Annotierungsvorschrift aus der Speichereinheit erfolgt typischerweise unabhängig vom Nutzer der medizinischen Bildgebungseinheit, insbesondere vom Bereitstellen der Planungsbildvorschrift für die Radiotherapieplanung. Die Speichereinheit kann beispielsweise mit dem Radiologieinformationssystem und/oder dem PACS-Bildarchivierungssystem verbunden sein, welche jeweils die Annotierungsvorschrift in die Speichereinheit beispielsweise auf Anweisung der Recheneinheit laden können, damit sie aus der Speichereinheit abgerufen werden kann. Grundsätzlich ist es denkbar, dass die Annotierungsvorschrift von einem Hersteller der medizinischen Bildgebungseinheit in die Speichereinheit insbesondere vor einem Bereitstellen der medizinischen Bildgebungseinheit in einem Krankenhaus geladen wird. Die Annotierungsvorschrift kann zusätzlich zur zweiten Rekonstruktionsvorschrift weitere Rekonstruktionsvorschriften aufweisen, wobei mittels der weiteren Rekonstruktionsvorschriften weitere medizinische Bilddatensätze rekonstruiert werden, welche für das Annotieren gemäß den Annotierungsinstruktionen verwenden können, wobei typischerweise die Annotierungsbildinformationen ermittelt werden.

Das Bereitstellen der Planungsbildvorschrift kann ein Vorgeben der Planungsbildvorschrift, insbesondere ein Vorgeben zumindest eines und/oder mehrerer Parameter der Aufnahmevorschrift und/oder zumindest eines und/oder mehrerer Rekonstruktionsparameter der ersten Rekonstruktionsvorschrift umfassen. Die Aufnahmevorschrift umfasst beispielsweise einen Röhrenstrom, eine Röhrenspannung, einen Messbereich und/oder eine Messdauer. Die Aufnahmevorschrift kann alternativ oder zusätzlich eine Rotationsgeschwindigkeit, einen Tischvorschub während der bildgebenden Messung, einen Tischvorschub vor und/oder nach der bildgebenden Messung, einen Pitch bei einer spiralen bildgebenden Messung und/oder eine Kollimierung von Röntgenstrahlen umfassen. Die Aufnahmevorschrift parametrisiert insbesondere die bildgebenden Messung, während welcher typischerweise die Rohdaten des Patienten erfasst werden.

Grundsätzlich ist es denkbar, dass die Planungsbildvorschrift eine physiologische Patientenzustandsvorschrift aufweist, wobei die physiologische Patientenzustandsvorschrift eine Herzphase und/oder eine Atemphase des Patienten aufweist und beim Rekonstruieren des ersten medizinischen Bilddatensatzes und beim Rekonstruieren des zweiten medizinischen Bilddatensatzes derart berücksichtigt wird, dass der erste medizinische Bilddatensatz und der zweite medizinische Bilddatensatz die physiologische Patientenzustandsvorschrift aufweisen. Dies ist insbesondere vorteilhaft, weil typischerweise eine Deformation des Patienten von dem physiologischen Patientenzustand abhängt, wodurch in diesem Fall ein Aufwand beim Kombinieren des ersten medizinischen Bilddatensatzes mit den Annotierungsbildinformationen verringert sein kann.

Die erste Rekonstruktionsvorschrift und die zweite Rekonstruktionsvorschrift unterscheiden sich typischerweise in zumindest einem Rekonstruktionsparameter der folgenden Liste:
- in einer Schichtdicke für die Rekonstruktion,
- in einem Schichtabstand für die Rekonstruktion,
- in einem Rekonstruktionskern,
- in einem Rekonstruktionsvolumen,
- in einem Bildwerteintervall,
- in einem Bildwertehistogramm,
- in einem iterativen Rekonstruktionsverfahren,
- in einem Artefaktkorrekturverfahren während der Rekonstruktion,
- in einer Abbildungstreue von Materialeigenschaften, welche bei der Rekonstruktion ermittelt wird.

Die erste Rekonstruktionsvorschrift und die zweite Rekonstruktionsvorschrift unterscheiden sich insbesondere derart, dass ein Bildeindruck des zweiten medizinischen Bilddatensatzes typischerweise im Vergleich zu einem Bildeindruck des ersten medizinischen Bilddatensatzes für den Nutzer ungeeignet erscheint. Der zweite medizinische Bilddatensatz kann für das Annotieren optimiert sein.

Das Bildwertehistogramm umfasst insbesondere eine Häufigkeitsverteilung der Bildwerte, beispielsweise in dem Zielvolumen, des ersten medizinischen Bilddatensatzes und/oder des zweiten medizinischen Bilddatensatzes.

Beispielsweise kann das Bildwerteintervall des zweiten medizinischen Bilddatensatzes größer sein als das Bildwerteintervall des ersten medizinischen Bilddatensatzes, weil das Bildwerteintervall des ersten medizinischen Bilddatensatzes vorzugsweise den Bildeindruck für den Nutzer wiedergibt, welchen der Nutzer typischerweise für die Radiotherapieplanung, insbesondere im Planungsbild, erwartet. Der erste medizinische Bilddatensatz bildet insbesondere das Planungsbild. Das Annotieren erfolgt vorzugsweise automatisch, beispielsweise in der Recheneinheit, bei welcher der Bildeindruck für den Nutzer typischerweise keine Rolle spielt.

In dem iterativen Rekonstruktionsverfahren kann sich die erste Rekonstruktionsvorschrift und die zweite Rekonstruktionsvorschrift derart unterscheiden, dass die erste Rekonstruktionsvorschrift faltungsbasiert, insbesondere ohne iterative Rekonstruktion, erfolgt und die zweite Rekonstruktionsvorschrift mit iterativer Rekonstruktion erfolgt oder umgekehrt. Alternativ oder zusätzlich kann sich ein iterativer Rekonstruktionsparameter des iterativen Rekonstruktionsverfahrens unterscheiden, insbesondere in einem Bildraumfilter der iterativen Rekonstruktion derart, dass typischerweise der Bildraumfilter der zweiten Rekonstruktionsvorschrift räumlich veränderliche Eigenschaften im Gegensatz zur ersten Rekonstruktionsvorschrift aufweist oder umgekehrt, und/oder in einer Regularisierung der iterativen Rekonstruktion. Beispielsweise kann die Regularisierung des iterativen Rekonstruktionsverfahrens der zweiten Rekonstruktionsvorschrift derart ausgebildet sein, dass ein Rauschen im zweiten medizinischen Bilddatensatz vorzugsweise nicht-linear unterdrückt wird. In diesem Fall kann die zweite Rekonstruktionsvorschrift derart ausgebildet sein, dass eine Optimierung des zweiten medizinischen Bilddatensatzes für das Annotieren auf Kosten einer Detektionswahrscheinlichkeit einer besonders kleinen anatomischen Struktur erfolgt. Alternativ kann die zweite Rekonstruktionsvorschrift derart ausgebildet sein, dass insbesondere mittels der Regularisierung die besonders kleine anatomische Struktur vorzugsweise präzise annotiert werden kann. Das iterative Rekonstruktionsverfahren umfasst insbesondere ein Rauschreduktionsverfahren und/oder einen nicht-linearen Bildfilter. Ob die besonders kleine anatomische Struktur nicht oder präzise erfasst wird, kann von der klinischen Fragestellung abhängen.

Das Erfassen der Rohdaten des Patienten erfolgt typischerweise bei der bildgebenden Messung. Für das Erfassen der Rohdaten wird die bildgebende Messung insbesondere einmalig durchgeführt. Die Rohdaten sind beispielsweise Projektionsdaten, wenn die medizinische Bildgebungseinheit einen Computertomographen aufweist, oder k-Raum-Daten, wenn die medizinische Bildgebungseinheit einen Magnetresonanztomographen aufweist. Es ist denkbar, dass die medizinische Bildgebungseinheit eine Kombination des Computertomographen, des Magnetresonanztomographen, eines Positronen-Emission-Tomographen und/oder eines Single-Photon-Emission-Computertomographen aufweist.

Die Rohdaten können beispielsweise nach dem Erfassen in dem Arbeitsspeicher und/oder dem Zwischenspeicher zwischengespeichert und/oder in das Radiologieinformationssystem und/oder in das PACS-Bildarchivierungssystem übertragen werden. Das Erfassen der Rohdaten kann eine Injektion eines Kontrastmittels in den Patienten umfassen. Die Rohdaten bilden insbesondere den Messbereich ab. Der Messbereich kann einem maximalen Gesichtsfeld entsprechen, welches typischerweise eine tunnelförmige Öffnung der medizinischen Bildgebungseinheit umfasst. Der Messbereich kann alternativ derart definiert sein, dass vorzugsweise für jeden Punkt innerhalb des Messbereichs Rohdaten über einen Winkelbereich von mindestens 180° erfasst werden können. Der Messbereich kann das maximale Gesichtsfeld ganz oder teilweise auffüllen. Der Messbereich ist typischerweise im Vergleich zum Rekonstruktionsvolumen der ersten Rekonstruktionsvorschrift und/oder zum Rekonstruktionsvolumen der zweiten Rekonstruktionsvorschrift kleiner oder gleich groß oder größer. Insbesondere für die Radiotherapieplanung kann der Messbereich kleiner sein, weil die erste Rekonstruktionsvorschrift und/oder die zweite Rekonstruktionsvorschrift derart ausgebildet sind, die Rohdaten in einem Bereich außerhalb des Messbereichs zu extrapolieren. Die Rohdaten werden üblicherweise nicht dem Nutzer auf der Anzeigeeinheit bereitgestellt.

Das Rekonstruieren des ersten medizinischen Bilddatensatzes und/oder des zweiten medizinischen Bilddatensatzes erfolgt üblicherweise in der Recheneinheit der medizinischen Bildgebungseinheit, beispielsweise in einer beliebigen Reihenfolge oder parallel. Der erste medizinische Bilddatensatz und/oder der zweite medizinische Bilddatensatz und/oder der annotierte medizinische Bilddatensatz können in das Radiologieinformationssystem und/oder das PACS-Bildarchivierungssystem übertragen und/oder auf der Anzeigeeinheit bereitgestellt werden. Der erste medizinische Bilddatensatz und/oder der zweite medizinische Bilddatensatz und/oder der annotierte medizinische Bilddatensatz weisen zumindest eine Bilddatensatzdimension auf. Es ist denkbar, dass vier Bilddatensatzdimensionen den Messbereich mit drei Bilddatensatzdimensionen und eine zeitliche Dimension als vierte Bilddatensatzdimension beschreiben.

Das Annotieren kann ein Aggregieren von funktionalen Informationen des Patienten umfassen. Das Annotieren basiert insbesondere auf einen Kontrast im zweiten medizinischen Bilddatensatz. Das Annotieren kann unter Berücksichtigung der absoluten Pixelwerte und/oder der Pixelwerte relativ zueinander unter Verwendung des zweiten medizinischen Bilddatensatzes erfolgen. Die Annotierungsinstruktionen sind insbesondere derart ausgebildet, dass das Annotieren ein Segmentieren, ein Klassifizieren und/oder ein Identifizieren im zweiten medizinischen Bilddatensatz umfasst. Das Segmentieren beschreibt insbesondere ein Abgrenzen, das Klassifizieren typischerweise ein Zuordnen in Klassen und/oder das Identifizieren üblicherweise ein Erkennen. Die Klassen können beispielsweise zwischen vitalem Gewebe, krankem Gewebe, funktionalem Gewebe, neovaskulärem Gewebe und/oder nekrotischem Gewebe unterscheiden. Das Identifizieren einer Tumor-Art ist insbesondere in Hinblick auf die Auswahl des Radiotherapieverfahrens vorteilhaft, weil Resistenzen gegenüber der ionisierenden Strahlung von der Tumor-Art abhängen können. Das Annotieren kann insbesondere eine Morphologie und/oder eine Anatomie und/oder die klinisch-relevante Struktur des Patienten und/oder das Implantat und/oder die anatomische Landmarke betreffen. Der Tumor kann insbesondere mehrere Metastasen umfassen. Die klinische-relevante Struktur kann insbesondere einen Lungenknoten und/oder einen Lymphknoten beschreiben. Alternativ oder zusätzlich kann das Annotieren ein Modellieren der Anatomie, der klinisch-relevanten Struktur des Patienten, des Implantats und/oder der anatomischen Landmarke in einem mathematisch parametrisierbaren Formmodell umfassen. Das Modellieren umfasst insbesondere ein Minimieren eines Abstandsmaßes.

Grundsätzlich ist es denkbar, dass das Annotieren ein Auswählen eines annotierten medizinischen Bilddatensatzes beispielsweise aus dem Radiologieinformationssystem und/oder dem PACS-Bildarchivierungssystem umfasst, welcher bei einem Vergleich eine hohe Ähnlichkeit zu dem zweiten medizinischen Bilddatensatz aufweist. In diesem Fall werden typischerweise Annotierungsbildinformationen des ausgewählten annotierten medizinischen Bilddatensatzes auf den zweiten medizinischen Bilddatensatz registriert, wobei die Annotierungsbildinformationen des zweiten medizinischen Bilddatensatzes ermittelt werden.

Das Annotieren des zweiten medizinischen Bilddatensatzes kann ein Extrahieren von Radiomics-Merkmalen umfassen. Die Radiomics-Merkmale umfassen insbesondere ein aus dem zweiten medizinischen Bilddatensatz extrahiertes Maß, insbesondere eine Form, eine Bildwertverteilung und/oder eine Textur der Pixelwerte insbesondere des Zielvolumens und/oder des Gewebes außerhalb des Zielvolumens.

Die Annotierungsinstruktionen umfassen insbesondere Bildverarbeitungsinstruktionen und/oder können insbesondere in Annotierungsprogrammcodemitteln abgebildet werden. Die Annotierungsinstruktionen umfassen insbesondere einen Mustererkennungs- und/oder einen Bilderkennungsalgorithmus. Das Annotieren erfolgt beispielsweise in der Recheneinheit, in welcher die Annotierungsprogrammcodemittel ausgeführt werden können.

Die Annotierungsbildinformationen liegen typischerweise in einer Datenstruktur vor. Die Annotierungsbildinformationen umfassen insbesondere eine Binärmaske, eine Wahrscheinlichkeitsmaske, eine Gitternetzstruktur, eine Polygonlinie, Eckpunkte eines Vielecks und/oder eine Beschriftung. Die Annotierungsbildinformationen liegen typischerweise relativ zu einem Koordinatensystem des zweiten medizinischen Bilddatensatzes vor und/oder können schichtweise sortiert werden.

Der zweite medizinische Bilddatensatz wird typischerweise nach dem Ermitteln der Annotierungsbildinformationen, insbesondere nach dem Erzeugen des annotierten medizinischen Bilddatensatzes, verworfen. In anderen Worten weist der erzeugte annotierte medizinische Bilddatensatz typischerweise den ersten medizinischen Bilddatensatz, die Annotierungsbildinformationen und nicht den zweiten medizinischen Bilddatensatz auf.

Das Kombinieren umfasst beispielsweise ein Übertragen der Annotierungsbildinformationen von dem zweiten medizinischen Bilddatensatz auf den ersten medizinischen Bilddatensatz insbesondere mittels der Recheneinheit, wobei typischerweise ein Koordinatensystem des ersten medizinischen Bilddatensatzes und ein Koordinatensystem des zweiten medizinischen Bilddatensatzes aufeinander abgestimmt werden können. Das Kombinieren umfasst insbesondere ein Registrieren und/oder ein Modellieren und/oder ein Interpolieren und/oder ein Extrapolieren des ersten medizinischen Bilddatensatzes und/oder der Annotierungsbildinformationen und/oder des zweiten medizinischen Bilddatensatzes mit den Annotierungsbildinformationen.

Der annotierte medizinische Bilddatensatz liegt beispielsweise in einem DICOM-Bildformat vor. Der erste medizinische Bilddatensatz wird beispielsweise als Bildinformation des DICOM-Bildformats abgespeichert und/oder die Annotierungsbildinformationen, insbesondere die Datenstruktur, wird als Dateninformation des DICOM-Bildformats abgespeichert. Grundsätzlich ist es denkbar, dass die Annotierungsbildinformationen zusätzlich zu dem ersten medizinischen Bilddatensatz als die Bildinformation des DICOM-Bildformats abgespeichert werden. Beispielsweise können die Annotierungsbildinformationen über dem ersten medizinischen Bilddatensatz eingeblendet werden. Das Erzeugen des annotierten medizinischen Bilddatensatzes umfasst insbesondere ein Erzeugen eines segmentierten medizinischen Bilddatensatzes, eines klassifizierten medizinischen Bilddatensatzes, eines identifizierten medizinischen Bilddatensatzes und/oder eines modellierten medizinischen Bilddatensatzes.

Der annotierte medizinische Bilddatensatz liegt beispielsweise in einer DICOM-Bildformat-Datei vor. Es ist denkbar, dass der annotierte medizinische Bilddatensatz mehrere Dateien umfasst, wobei beispielsweise eine erste Datei den ersten medizinischen Bilddatensatz und eine zweite Datei die Annotierungsbildinformationen aufweist. Die erste Datei und/oder die zweite Datei können in dem DICOM-Bildformat und/oder in einem weiteren Bildformat vorliegen. Grundsätzlich ist es denkbar, dass für jede Schicht, beispielsweise für jedes medizinische Bild mit zwei Bilddatensatzdimensionen, des annotierten medizinischen Bilddatensatzes eine Datei mit dem schichtbezogenen Planungsbild sowie mit den schichtbezogenen Annotierungsbildinformationen oder für jede Schicht zwei Dateien, jeweils mit dem schichtbezogenen Planungsbild und mit den schichtbezogenen Annotierungsbildinformationen, erstellt werden.

Der annotierte medizinische Bilddatensatz kann beispielsweise auf einem Datenträger und/oder auf der Anzeigeeinheit und/oder in dem Radiologieinformationssystem und/oder in dem PACS-Bildarchivierungssystem und/oder auf dem Radiotherapieplanungssystem bereitgestellt werden. Das Radiotherapieplanungssystem kann die Anzeigeeinheit und/oder die Eingabemittel umfassen. Das Radiotherapieplanungssystem kann ein Teil der medizinischen Bildgebungseinheit sein oder außerhalb der medizinischen Bildgebungseinheit angeordnet sein. Die medizinische Bildgebungseinheit ist beispielsweise über ein Netzwerk und/oder das Radiologieinformationssystem und/oder das PACS-Bildarchivierungssystem mit dem Radiotherapieplanungssystem verbunden. Die Anzeigeeinheit kann als Teil einer Arbeitsstation zu einer Bildverarbeitung ausgebildet sein. Die Radiotherapieplanung und/oder die Radiotherapie erfolgen typischerweise nach dem Bereitstellen des annotierten medizinischen Bilddatensatzes.

Eine Ausführungsform sieht vor, dass sich ein Artefaktkorrekturverfahren der ersten Rekonstruktionsvorschrift von einem Artefaktkorrekturverfahren der zweiten Rekonstruktionsvorschrift derart unterscheidet, dass nach der Anwendung des Artefaktkorrekturverfahrens eine Anzahl an Bildartefakten in dem zweiten medizinischen Bilddatensatz geringer ist als eine Anzahl an Bildartefakten in dem ersten medizinischen Bilddatensatz. Das Artefaktkorrekturverfahren umfasst insbesondere ein Verfahren zur Reduzierung von Strahlaufhärtungs- und/oder Metallartefakten. Das Artefaktkorrekturverfahren der ersten Rekonstruktionsvorschrift kann insbesondere von dem Nutzer beim Bereitstellen der Planungsbildvorschrift abgewählt werden. Das Anwenden des Artefaktkorrekturverfahrens, insbesondere der ersten Rekonstruktionsvorschrift, kann den Bildeindruck für den Nutzer verfälschen. Vorzugsweise kann ohne Rücksicht auf den Bildeindruck für den Nutzer die Anzahl an Bildartefakten in dem zweiten medizinischen Bilddatensatz im Vergleich zum ersten medizinischen Bilddatensatz reduziert werden.

Eine Ausführungsform sieht vor, dass eine Abbildungstreue von Materialeigenschaften der ersten Rekonstruktionsvorschrift höher ist als eine Abbildungstreue der Materialeigenschaften der zweiten Rekonstruktionsvorschrift. Vorzugsweise bildet der erste medizinische Bilddatensatz die Materialeigenschaften präziser ab als der zweite medizinische Bilddatensatz. Vorteilhafterweise kann der erste medizinische Bilddatensatz, insbesondere das Planungsbild, die Materialeigenschaften präziser als der zweite medizinische Bilddatensatz abbilden.

Eine Ausführungsform sieht vor, dass eine Schichtdicke der zweiten Rekonstruktionsvorschrift geringer ist als eine Schichtdicke der ersten Rekonstruktionsvorschrift. Typischerweise kann ein Informationsverlust, beispielsweise aufgrund einer unvorteilhaften Erfüllung eines Abtasttheorems, verhindert werden. Typischerweise wählt der Nutzer als Schichtdicke der ersten Rekonstruktionsvorschrift beim Bereitstellen der Planungsbildvorschrift eine vergleichsweise große Schichtdicke, beispielsweise 4 mm, während die Schichtdicke der zweiten Rekonstruktionsvorschrift geringer insbesondere 3 mm, vorteilhafterweise 2 mm, besonders vorteilhafterweise 1 mm ist.

Eine Ausführungsform sieht vor, dass ein Rekonstruktionsvolumen der zweiten Rekonstruktionsvorschrift größer ist als ein Rekonstruktionsvolumen der ersten Rekonstruktionsvorschrift. Das Rekonstruktionsvolumen der zweiten Rekonstruktionsvorschrift kann den Messbereich ganz oder teilweise auffüllen. Das Rekonstruktionsvolumen der zweiten Rekonstruktionsvorschrift kann beispielsweise das Zielvolumen und/oder das Risikoorgan ganz umfassen, während das Rekonstruktionsvolumen der ersten Rekonstruktionsvorschrift insbesondere durch den Nutzer derart festgelegt wird, dass insbesondere an Randpositionen Informationen verloren gehen können. Beispielsweise ist es für das Annotieren vorteilhafter, wenn das Rekonstruktionsvolumen das Zielvolumen und/oder das Risikoorgan ganz umfasst. Das Rekonstruktionsvolumen der zweiten Rekonstruktionsvorschrift kann vorzugsweise mehr als 5%, vorteilhafterweise 10% und/oder besonders vorteilhafterweise 15% größer sein als das Rekonstruktionsvolumen der ersten Rekonstruktionsvorschrift.

Eine Ausführungsform sieht vor, dass sich die erste Rekonstruktionsvorschrift derart von der zweiten Rekonstruktionsvorschrift unterscheidet, dass eine Bildauflösung in dem zweiten medizinischen Bilddatensatz höher ist als eine Bildauflösung in dem ersten medizinischen Bilddatensatz. In diesem Fall kann vorzugsweise das Annotieren präziser erfolgen, weil der zweite medizinische Bilddatensatz vorzugsweise mehr Bildinformationen aufweist als der erste medizinische Bilddatensatz, bei welchem typischerweise wegen der verringerten Bildauflösung Bildinformationen gemittelt sein können. Die Bildauflösung des zweiten medizinischen Bilddatensatzes kann um einen Faktor 1,05, vorteilhafterweise um einen Faktor 2 und besonders vorteilhafterweise um einen Faktor mehr als 4 größer sein als die Bildauflösung des ersten medizinischen Bilddatensatzes.

Eine Ausführungsform sieht vor, dass sich ein Filter der zweiten Rekonstruktionsvorschrift derart von einem Filter der ersten Rekonstruktionsvorschrift unterscheidet, dass Bildkanten in dem zweiten medizinischen Bilddatensatz konturierter abgebildet sind als Bildkanten in dem ersten medizinischen Bilddatensatz. Der Filter der zweiten Rekonstruktionsvorschrift ist insbesondere derart optimiert, dass in dem zweiten medizinischen Bilddatensatz insbesondere anatomische Kanten und/oder morphologische Kanten besser hervorgehoben werden können. Die Bildkanten können insbesondere die anatomischen Kanten und/oder die morphologischen Kanten abbilden. Der Filter der zweiten Rekonstruktionsvorschrift kann insbesondere einen Medianfilter aufweisen. Der Filter der ersten Rekonstruktionsvorschrift kann insbesondere einen Mittelwertfilter aufweisen, welcher typischerweise den Bildeindruck für den Nutzer weniger verfälscht. Der Filter beschreibt insbesondere ein Optimieren von Bildwerten und/oder von Bildrauschen. Die Bildkanten werden insbesondere dann konturierter abgebildet, wenn diejenigen Pixelwerte, welche die Bildkanten abbilden, relativ zueinander einen größeren Abstand aufweisen.

Eine Ausführungsform sieht vor, dass die zweite Rekonstruktionsvorschrift Referenzwertvorgaben aufweist, wobei beim Rekonstruieren des zweiten medizinischen Bilddatensatzes die Referenzwertvorgaben berücksichtigt werden, wodurch sich ein Kontrast des ersten medizinischen Bilddatensatzes von einem Kontrast des zweiten medizinischen Bilddatensatzes unterscheidet. Die Referenzwertvorgaben ermöglichen insbesondere das Standardisieren des Bereitstellens der Annotierungsbildinformationen, wodurch insbesondere die standardisierten Annotierungsbildinformationen bereitgestellt werden. Die Referenzwertvorgaben sind insbesondere für die automatisierte Radiotherapieplanung vorteilhaft. Beispielsweise können die Referenzwertvorgaben vorgeben, dass der zweite medizinische Bilddatensatz einen Bildeindruck einer anderen Röhrenspannung aufweist, als die Röhrenspannung ermöglichen kann, welche beim Erfassen der Rohdaten verwendet wird. Der Kontrast umfasst insbesondere eine Darstellung der Bildkanten in Pixelwerten. Der Kontrast hängt insbesondere von einer spektralen Gewichtung der Rohdaten ab.

Eine Ausführungsform sieht vor, dass die Referenzwertvorgaben eine CT-Zahl für ein Organ des Patienten aufweisen. Die Referenzwertvorgaben können beispielsweise einem imaginären Referenzpatienten entsprechen. In dieser Ausführungsform kann beispielsweise der zweite medizinische Bilddatensatz derart rekonstruiert werden, dass das Organ des Patienten die CT-Zahl aufweist und/oder eine geringe Abweichung von der CT-Zahl aufweist, was insbesondere für die automatische Radiotherapieplanung vorteilhaft sein kann. Das Organ des Patienten kann beispielsweise eine Leber des Patienten sein, wobei in diesem Fall die CT-Zahl zwischen 50 und 100 HU, vorteilhafterweise zwischen 70 und 90 HU, vorzugsweise zwischen 75 und 85 HU und/oder besonders vorzugsweise bei 80 HU liegen kann.

Eine Ausführungsform sieht vor, dass die Aufnahmevorschrift eine Mehr-Energie-Aufnahmevorschrift für ein Erfassen von spektral aufgelösten Rohdaten aufweist und wobei sich die erste Rekonstruktionsvorschrift von der zweiten Rekonstruktionsvorschrift in einer Gewichtung der spektral aufgelösten Rohdaten unterscheiden. Die Mehr-Energie-Aufnahmevorschrift kann eine Dual-Energie Messung umfassen. Beispielsweise kann die zweite Rekonstruktionsvorschrift derart ausgebildet sein, dass das Annotieren aufgrund der Gewichtung der spektral aufgelösten Rohdaten insbesondere bessere Ergebnisse liefert.

Eine Ausführungsform sieht vor, dass sich die erste Rekonstruktionsvorschrift derart von der zweiten Rekonstruktionsvorschrift unterscheidet, dass der erste medizinische Bilddatensatz und der zweite medizinische Bilddatensatz unterschiedliche Bilddatensatzdimensionen aufweist. Beispielsweise kann es für das Annotieren vorteilhaft sein, wenn der zweite medizinische Bilddatensatz alle Bilddatensatzdimensionen verwendet, während der erste medizinische Bilddatensatz beispielsweise eine verringerte Anzahl an Bilddatensatzdimensionen aufweist. Beispielsweise kann der zweite medizinische Bilddatensatz die zeitliche Dimension aufweisen, während der erste medizinische Bilddatensatz beispielsweise einen Mittelwert über die zeitliche Dimension aufweist, wodurch in diesem Beispiel der erste medizinische Bilddatensatz weniger Bilddatensatzdimensionen aufweist als der zweite medizinische Bilddatensatz. Das Annotieren, insbesondere der klinisch-relevanten Struktur, kann vorzugsweise präziser mittels der zeitlichen Dimension erfolgen, insbesondere wenn die klinisch-relevante Struktur den Lungenknoten und/oder den Lymphknoten aufweist. Die klinisch-relevante Struktur kann in diesem Fall derart annotiert werden, dass Rohdaten bei einer Einatmung und einer Ausatmung des Patienten erfasst werden, wodurch die zeitliche Dimension gebildet wird.

Eine Ausführungsform sieht vor, dass der erste medizinische Bilddatensatz drei Bilddatensatzdimensionen und der zweite medizinische Bilddatensatz vier Bilddatensatzdimensionen aufweist. Diese Ausführungsform ist insofern vorteilhaft, dass für das Annotieren alle verfügbaren Bildinformationen verwendet werden können, insbesondere wenn die Rohdaten räumlich und zeitlich aufgelöst erfasst werden.

Eine Ausführungsform sieht vor, dass zusätzlich zur Planungsbildvorschrift eine Annotierungskennung bereitgestellt wird, wobei beim Annotieren des zweiten medizinischen Bilddatensatzes die Annotierungskennung zusätzlich zu den Annotierungsinstruktionen als Eingangsparameter eingehen, wobei die Annotierungsbildinformationen die Annotierungskennung aufweist und wobei die Annotierungskennung einen zu behandelnden Tumor des Patienten, ein Risikoorgan des Patienten, eine anatomische Landmarke des Patienten und/oder ein zu klassifizierendes Gewebe des Patienten aufweist. Die Annotierungskennung wird insbesondere durch den Nutzer beim Bereitstellen der Planungsbildvorschrift beispielsweise mittels der Anzeigeeinheit und/oder der Eingabemittel bereitgestellt. Die Annotierungskennung umfasst insbesondere eine Auswahl, welcher zu behandelnde Tumor, welches Risikoorgan, welche anatomische Landmarke und/oder welches zu klassifizierendes Gewebe des Patienten annotiert werden. Die Annotierungskennung gibt insbesondere die Anatomie, die klinisch-relevante Struktur des Patienten, das Implantat und/oder die anatomische Landmarke für das Annotieren vor. Die Annotierungskennung umfasst insbesondere die klinische Fragestellung, welche beim Annotieren vorzugsweise berücksichtigt werden kann. Die Annotierungsvorschrift, insbesondere die Annotierungsinstruktionen und/oder die zweite Rekonstruktionsvorschrift können insbesondere von der klinischen Fragestellung abhängen.

Eine Ausführungsform sieht vor, dass das Annotieren des zweiten medizinischen Bilddatensatzes unter Verwendung eines künstlichen neuronalen Netzes erfolgt. Das künstliche neuronale Netz kann insbesondere auf den Bildeindruck des zweiten medizinischen Bilddatensatz trainiert sein, welcher typischerweise im Vergleich zum Bildeindruck des ersten medizinischen Bilddatensatz verfälscht sein kann. Das künstliche neuronale Netz ermöglicht vorzugsweise ein schnelleres Annotieren in diesem Fall. Das künstliche neuronale Netz weist typischerweise eine Eingangsschicht und eine Ausgangsschicht auf, wobei üblicherweise die Eingangsschicht und die Ausgangsschicht über gewichtete Verbindungen verbunden sind. Die gewichteten Verbindungen können gewichtete Kanten zwischen Knoten in der Eingangsschicht, in der Ausgangsschicht und/oder in versteckten Schichten zwischen der Eingangsschicht und der Ausgangsschicht aufweisen. Das künstliche neuronale Netz kann ein rekurrentes neuronales Netz sein, welches gemäß den Annotierungsbildinformationen weiterentwickelt und/oder verbessert werden kann. Das künstliche neuronale Netz wird typischerweise vor dem Annotieren des zweiten medizinischen Bilddatensatzes unter Verwendung weiterer medizinischer Trainingsbilddatensätze und weiterer Trainingsannotierungsbildinformationen trainiert. Das Annotieren unter Verwendung des künstlichen neuronalen Netzes erfolgt typischerweise derart, dass an der Eingangsschicht der zweite anatomische Bilddatensatz und/oder die Annotierungsinstruktionen bereitgestellt werden, wobei an der Ausgangsschicht die Annotierungsbildinformationen bereitliegen. Grundsätzlich ist es denkbar, dass die Annotierungsinstruktionen in den gewichteten Verbindungen abgebildet werden. Das trainierte künstliche neuronale Netz kann beispielsweise in der Speichereinheit und/oder im Zwischenspeicher und/oder im Arbeitsspeicher insbesondere vor dem Annotieren abgespeichert werden.

Eine erfindungsgemäße medizinische Bildgebungseinheit weist eine Recheneinheit auf, wobei die medizinische Bildgebungseinheit nach dem Verfahren für das Bereitstellen des annotierten medizinischen Bilddatensatzes für die Radiotherapieplanung des Patienten ausgebildet ist. Die Recheneinheit weist typischerweise einen Prozessor, eine Netzwerkverbindung, den Arbeitsspeicher und/oder den Zwischenspeicher auf.

Ein erfindungsgemäßes Computerprogrammprodukt, welches direkt in einen Speicher der Recheneinheit ladbar ist, weist Programmcodemittel auf, um das Verfahren für das Bereitstellen des annotierten medizinischen Bilddatensatzes für die Radiotherapieplanung des Patienten auszuführen, wenn das Computerprogrammprodukt in der Recheneinheit ausgeführt wird.

Das Computerprogrammprodukt kann ein Computerprogramm sein oder ein Computerprogramm umfassen. Das Computerprogrammprodukt weist insbesondere die Programmcodemittel auf, welche die erfindungsgemäßen Verfahrensschritte abbilden. Dadurch kann das erfindungsgemäße Verfahren definiert und wiederholbar ausgeführt sowie eine Kontrolle über eine Weitergabe des erfindungsgemäßen Verfahrens ausgeübt werden. Das Computerprogrammprodukt ist vorzugsweise derart konfiguriert, dass die Recheneinheit mittels des Computerprogrammprodukts die erfindungsgemäßen Verfahrensschritte ausführen kann. Die Programmcodemittel können insbesondere in einen Speicher der Recheneinheit geladen und typischerweise mittels eines Prozessors der Recheneinheit mit Zugriff auf den Speicher ausgeführt werden. Wenn das Computerprogrammprodukt, insbesondere die Programmcodemittel, in der Recheneinheit ausgeführt wird, können typischerweise alle erfindungsgemäßen Ausführungsformen des beschriebenen Verfahrens durchgeführt werden. Das Computerprogrammprodukt ist beispielsweise auf einem physischen, computerlesbaren Medium gespeichert und/oder digital als Datenpaket in einem Computernetzwerk hinterlegt. Das Computerprogrammprodukt kann das physische, computerlesbare Medium und/oder das Datenpaket in dem Computernetzwerk darstellen. So kann die Erfindung auch von dem physischen, computerlesbaren Medium und/oder dem Datenpaket in dem Computernetzwerk ausgehen. Das physische, computerlesbare Medium ist üblicherweise unmittelbar mit der Recheneinheit verbindbar, beispielsweise indem das physische, computerlesbare Medium in ein DVD-Laufwerk eingelegt oder in einen USB-Port gesteckt wird, wodurch die Recheneinheit auf das physische, computerlesbare Medium insbesondere lesend zugreifen kann. Das Datenpaket kann vorzugsweise aus dem Computernetzwerk abgerufen werden. Das Computernetzwerk kann die Recheneinheit aufweisen oder mittels einer Wide-Area-Network- (WAN) bzw. einer (Wireless-)Local-Area-Network-Verbindung (WLAN oder LAN) mit der Recheneinheit mittelbar verbunden sein. Beispielsweise kann das Computerprogrammprodukt digital auf einem Cloud-Server an einem Speicherort des Computernetzwerks hinterlegt sein, mittels des WAN über das Internet und/oder mittels des WLAN bzw. LAN auf die Recheneinheit insbesondere durch das Aufrufen eines Downloadlinks, welcher zu dem Speicherort des Computerprogrammprodukts verweist, übertragen werden.

Bei der Beschreibung der Vorrichtung erwähnte Merkmale, Vorteile oder alternative Ausführungsformen sind ebenso auf das Verfahren zu übertragen und umgekehrt. Mit anderen Worten können Ansprüche auf das Verfahren mit Merkmalen der Vorrichtung weitergebildet sein und umgekehrt. Insbesondere kann die erfindungsgemäße Vorrichtung in dem Verfahren verwendet werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 ein Verfahren für ein Bereitstellen eines annotierten medizinischen Bilddatensatzes für eine Radiotherapieplanung eines Patienten in einem ersten Ausführungsbeispiel,
Fig. 2 das Verfahren in einem zweiten Ausführungsbeispiel und
Fig. 3 eine medizinische Bildgebungseinheit in einem dritten Ausführungsbeispiel.

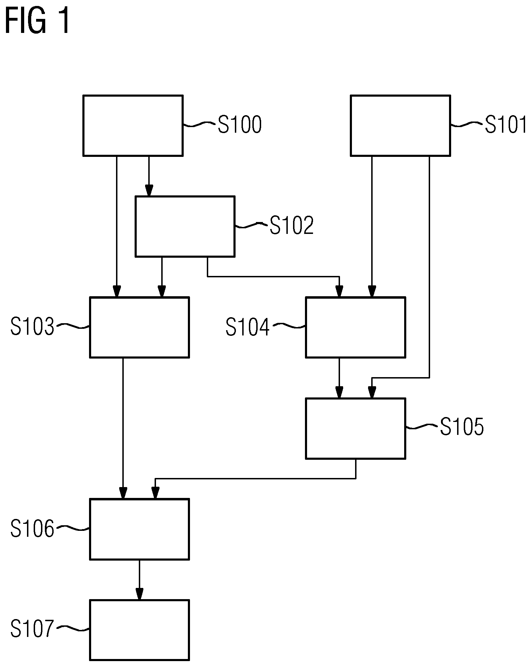

**Fig. 1** zeigt ein Flussdiagramm des Verfahrens für ein Bereitstellen eines annotierten medizinischen Bilddatensatzes für eine Radiotherapieplanung eines Patienten in einem ersten Ausführungsbeispiel.

Verfahrensschritt S100 kennzeichnet ein Bereitstellen einer Planungsbildvorschrift für die Radiotherapieplanung, welche eine Aufnahmevorschrift und eine erste Rekonstruktionsvorschrift aufweist.

Verfahrensschritt S101 kennzeichnet ein Abrufen einer Annotierungsvorschrift aus einer Speichereinheit, wobei die Annotierungsvorschrift Annotierungsinstruktionen und eine zweite Rekonstruktionsvorschrift aufweist und wobei sich die zweite Rekonstruktionsvorschrift von der ersten Rekonstruktionsvorschrift unterscheidet.

Verfahrensschritt S102 kennzeichnet ein Erfassen von Rohdaten des Patienten mittels einer medizinischen Bildgebungseinheit gemäß der Aufnahmevorschrift.

Verfahrensschritt S103 kennzeichnet ein Rekonstruieren eines ersten medizinischen Bilddatensatzes gemäß der ersten Rekonstruktionsvorschrift unter Verwendung der Rohdaten.

Verfahrensschritt S104 kennzeichnet ein Rekonstruieren eines zweiten medizinischen Bilddatensatzes gemäß der zweiten Rekonstruktionsvorschrift unter Verwendung der Rohdaten.

Verfahrensschritt S105 kennzeichnet ein Annotieren des zweiten medizinischen Bilddatensatzes gemäß den Annotierungsinstruktionen, wobei Annotierungsbildinformationen ermittelt werden.

Verfahrensschritt S106 kennzeichnet ein Kombinieren des ersten medizinischen Bilddatensatzes mit den Annotierungsbildinformationen, wobei ein annotierter medizinischer Bilddatensatz erzeugt wird.

Verfahrensschritt S107 kennzeichnet ein Bereitstellen des annotierten medizinischen Bilddatensatzes für die Radiotherapieplanung des Patienten.

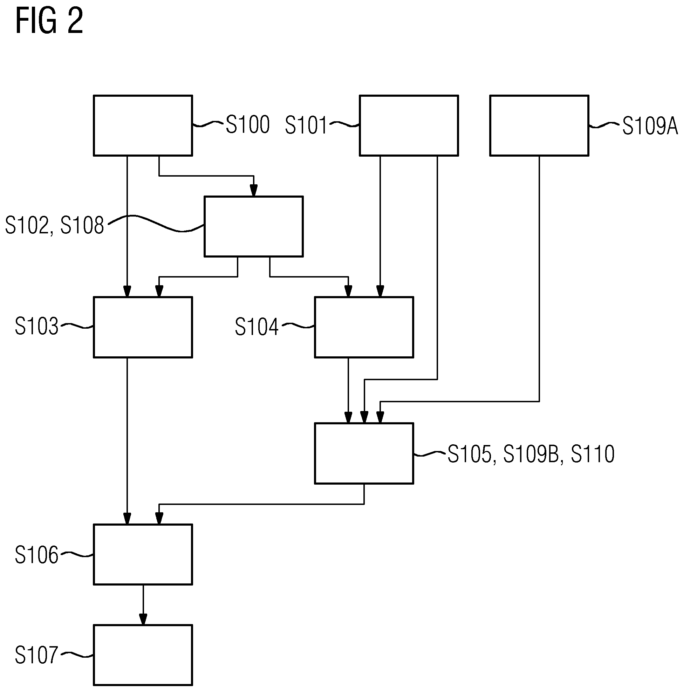

**Fig. 2** zeigt das Verfahren in einem zweiten Ausführungsbeispiel, wobei einer oder mehrere der im Vergleich zur Fig. 1 hinzugefügten Verfahrensschritte in weiteren Ausführungsbeispielen grundsätzlich weggelassen werden können.

Verfahrensschritt S108 kennzeichnet, dass die Aufnahmevorschrift eine Mehr-Energie-Aufnahmevorschrift für ein Erfassen von spektral aufgelösten Rohdaten aufweist, wobei sich die erste Rekonstruktionsvorschrift von der zweiten Rekonstruktionsvorschrift in einer Gewichtung der spektral aufgelösten Rohdaten unterscheiden.

Verfahrensschritt S109A kennzeichnet, dass zusätzlich zur Planungsbildvorschrift eine Annotierungskennung bereitgestellt wird.

Verfahrensschritt S109B kennzeichnet, dass beim Annotieren des zweiten medizinischen Bilddatensatzes die Annotierungskennung zusätzlich zu den Annotierungsinstruktionen als Eingangsparameter eingehen, wobei die Annotierungsbildinformationen die Annotierungskennung aufweist und wobei die Annotierungskennung einen zu behandelnden Tumor des Patienten, ein Risikoorgan des Patienten, eine anatomische Landmarke des Patienten und/oder ein zu klassifizierendes Gewebe des Patienten aufweist.

Verfahrensschritt S110 kennzeichnet, dass das Annotieren des zweiten medizinischen Bilddatensatzes unter Verwendung eines künstlichen neuronalen Netzes erfolgt.

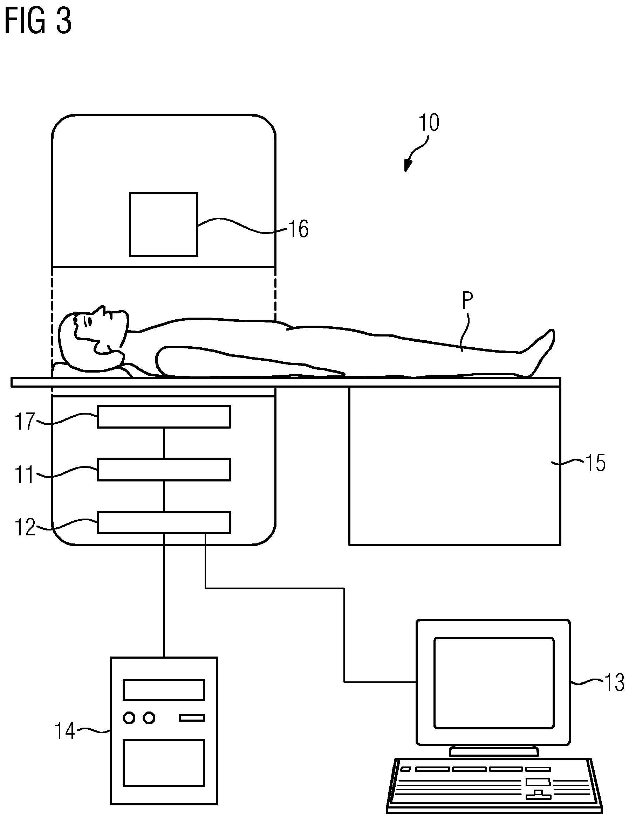

**Fig. 3** zeigt die medizinische Bildgebungseinheit 10 in einem dritten Ausführungsbeispiel. Die medizinische Bildgebungseinheit 10 weist die Recheneinheit 11, einen Zwischenspeicher 12 und eine Anzeigeeinheit 13 mit Eingabemitteln für einen Nutzer auf. Die medizinische Bildgebungseinheit 10, insbesondere der Zwischenspeicher 12, ist mit einer Speichereinheit 14 verbunden. Der Nutzer kann mittels der Anzeigeeinheit 13 eine Planungsbildvorschrift für eine Radiotherapieplanung bereitstellen. Die Planungsbildvorschrift und die Annotierungsvorschrift werden in dem Zwischenspeicher 12 gemeinsam, insbesondere bis zu einem Abruf für das Erfassen der Rohdaten und/oder für ein Rekonstruieren des ersten medizinischen Bilddatensatzes und/oder für ein Rekonstruieren des zweiten medizinischen Bilddatensatzes und/oder für ein Annotieren des zweiten medizinischen Bilddatensatzes zwischengespeichert. Der Zwischenspeicher 12 kann als ein Arbeitsspeicher der Recheneinheit 11 ausgebildet sein. Der Zwischenspeicher 12 und die Recheneinheit 11 können für ein Kombinieren des ersten medizinischen Bilddatensatzes mit den Annotierungsbildinformationen und/oder für ein Bereitstellen des annotierten medizinischen Bilddatensatzes ausgebildet sein. Die medizinische Bildgebungseinheit 10 ist zu einem Abrufen einer Annotierungsvorschrift aus der Speichereinheit 14 und dem Zwischenspeichern der Annotierungsvorschrift in dem Zwischenspeicher 12, insbesondere mittels der Recheneinheit 11, ausgebildet. Der Patient P ist auf einer Patientenliege 15 gelagert insbesondere zum Erfassen der Rohdaten. Der annotierte medizinische Bilddatensatz wird auf der Anzeigeeinheit 13 für die Radiotherapieplanung des Patienten P vorzugsweise von der Recheneinheit 11 und/oder dem Zwischenspeicher 12 bereitgestellt.

Ein Radiotherapiesystem zu einer Durchführung einer Radiotherapie gemäß der Radiotherapieplanung ist in Fig. 3 nicht gezeigt. Die Radiotherapie kann gemäß verschiedenen Radiotherapieverfahren erfolgen. Grundsätzlich ist es denkbar, dass die medizinische Bildgebungseinheit 10 das Radiotherapiesystem aufweist.

In diesem Ausführungsbeispiel ist die medizinische Bildgebungseinheit 10 als ein Computertomograph ausgebildet und weist eine Röntgenquelle 16 und einen Röntgendetektor 17 zum Erfassen der Rohdaten auf, welches typischerweise von der Recheneinheit gesteuert werden kann. Alternativ oder zusätzlich kann die medizinische Bildgebungseinheit 10 als ein Magnetresonanztomograph mit einer Hauptmagnetfeldeinheit und einer Gradientenmagnetfeldeinheit ausgebildet sein.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren für ein Bereitstellen eines annotierten medizinischen Bilddatensatzes für eine Radiotherapieplanung eines Patienten, mit folgenden Schritten:
- Bereitstellen einer Planungsbildvorschrift für die Radiotherapieplanung, welche eine Aufnahmevorschrift und eine erste Rekonstruktionsvorschrift aufweist,
- Abrufen einer Annotierungsvorschrift aus einer Speichereinheit, wobei die Annotierungsvorschrift Annotierungsinstruktionen und eine zweite Rekonstruktionsvorschrift aufweist und wobei sich die zweite Rekonstruktionsvorschrift von der ersten Rekonstruktionsvorschrift unterscheidet,
- Erfassen von Rohdaten des Patienten mittels einer medizinischen Bildgebungseinheit gemäß der Aufnahmevorschrift,
- Rekonstruieren eines ersten medizinischen Bilddatensatzes gemäß der ersten Rekonstruktionsvorschrift unter Verwendung der Rohdaten,
- Rekonstruieren eines zweiten medizinischen Bilddatensatzes gemäß der zweiten Rekonstruktionsvorschrift unter Verwendung der Rohdaten,
- Annotieren des zweiten medizinischen Bilddatensatzes gemäß den Annotierungsinstruktionen, wobei Annotierungsbildinformationen ermittelt werden,
- Kombinieren des ersten medizinischen Bilddatensatzes mit den Annotierungsbildinformationen, wobei ein annotierter medizinischer Bilddatensatz erzeugt wird, und
- Bereitstellen des annotierten medizinischen Bilddatensatzes für die Radiotherapieplanung des Patienten.

2. Verfahren nach Anspruch 1, wobei sich ein Artefaktkorrekturverfahren der ersten Rekonstruktionsvorschrift von einem Artefaktkorrekturverfahren der zweiten Rekonstruktionsvorschrift derart unterscheidet, dass nach der Anwendung des Artefaktkorrekturverfahrens eine Anzahl an Bildartefakten in dem zweiten medizinischen Bilddatensatz geringer ist als eine Anzahl an Bildartefakten in dem ersten medizinischen Bilddatensatz.

3. Verfahren nach einem der vorhergehenden Ansprüche, wobei eine Abbildungstreue von Materialeigenschaften der ersten Rekonstruktionsvorschrift höher ist als eine Abbildungstreue der Materialeigenschaften der zweiten Rekonstruktionsvorschrift.

4. Verfahren nach einem der vorhergehenden Ansprüche, wobei eine Schichtdicke der zweiten Rekonstruktionsvorschrift geringer ist als eine Schichtdicke der ersten Rekonstruktionsvorschrift.

5. Verfahren nach einem der vorhergehenden Ansprüche, wobei ein Rekonstruktionsvolumen der zweiten Rekonstruktionsvorschrift größer ist als ein Rekonstruktionsvolumen der ersten Rekonstruktionsvorschrift.

6. Verfahren nach einem der vorhergehenden Ansprüche, wobei sich die erste Rekonstruktionsvorschrift derart von der zweiten Rekonstruktionsvorschrift unterscheidet, dass eine Bildauflösung in dem zweiten medizinischen Bilddatensatz höher ist als eine Bildauflösung in dem ersten medizinischen Bilddatensatz.

7. Verfahren nach einem der vorhergehenden Ansprüche, wobei sich ein Filter der zweiten Rekonstruktionsvorschrift derart von einem Filter der ersten Rekonstruktionsvorschrift unterscheidet, dass Bildkanten in dem zweiten medizinischen Bilddatensatz konturierter abgebildet sind als Bildkanten in dem ersten medizinischen Bilddatensatz.

8. Verfahren nach einem der vorhergehenden Ansprüche, wobei die zweite Rekonstruktionsvorschrift Referenzwertvorgaben aufweist und wobei beim Rekonstruieren des zweiten medizinischen Bilddatensatzes die Referenzwertvorgaben berücksichtigt werden, wodurch sich ein Kontrast des ersten medizinischen Bilddatensatzes von einem Kontrast des zweiten medizinischen Bilddatensatzes unterscheidet.

9. Verfahren nach Anspruch 8, wobei die Referenzwertvorgaben eine CT-Zahl für ein Organ des Patienten aufweisen.

10. Verfahren nach einem der vorhergehenden Ansprüche, wobei die Aufnahmevorschrift eine Mehr-Energie-Aufnahmevorschrift für ein Erfassen von spektral aufgelösten Rohdaten aufweist und wobei sich die erste Rekonstruktionsvorschrift von der zweiten Rekonstruktionsvorschrift in einer Gewichtung der spektral aufgelösten Rohdaten unterscheiden.

11. Verfahren nach einem der vorhergehenden Ansprüche, wobei sich die erste Rekonstruktionsvorschrift derart von der zweiten Rekonstruktionsvorschrift unterscheidet, dass der erste medizinische Bilddatensatz und der zweite medizinische Bilddatensatz unterschiedliche Bilddatensatzdimensionen aufweist.

12. Verfahren nach Anspruch 11, wobei der erste medizinische Bilddatensatz drei Bilddatensatzdimensionen und der zweite medizinische Bilddatensatz vier Bilddatensatzdimensionen aufweist.

13. Verfahren nach einem der vorhergehenden Ansprüche, wobei zusätzlich zur Planungsbildvorschrift eine Annotierungskennung bereitgestellt wird, wobei beim Annotieren des zweiten medizinischen Bilddatensatzes die Annotierungskennung zusätzlich zu den Annotierungsinstruktionen als Eingangsparameter eingehen, wobei die Annotierungsbildinformationen die Annotierungskennung aufweist und wobei die Annotierungskennung einen zu behandelnden Tumor des Patienten, ein Risikoorgan des Patienten, eine anatomische Landmarke des Patienten und/oder ein zu klassifizierendes Gewebe des Patienten aufweist.

14. Verfahren nach einem der vorhergehenden Ansprüche, wobei das Annotieren des zweiten medizinischen Bilddatensatzes unter Verwendung eines künstlichen neuronalen Netzes erfolgt.

15. Medizinische Bildgebungseinheit, aufweisend eine Recheneinheit, wobei die medizinische Bildgebungseinheit nach einem der vorhergehenden Ansprüche ausgebildet ist.

16. Computerprogrammprodukt, welches direkt in einen Speicher einer Recheneinheit ladbar ist, mit Programmcodemitteln, um ein Verfahren nach einem der Ansprüche 1 bis 14 auszuführen, wenn das Computerprogrammprodukt in der Recheneinheit ausgeführt wird.
